(19) **Europäisches Patentamt**

**European Patent Office**

**Office européen des brevets**

(11) **EP 0 967 935 B1**

(12) **EUROPÄISCHE PATENTSCHRIFT**

(45) Veröffentlichungstag und Bekanntmachung des Hinweises auf die Patenterteilung:
**06.05.2004 Patentblatt 2004/19**

(21) Anmeldenummer: **98913567.8**

(22) Anmeldetag: **20.02.1998**

(51) Int Cl.⁷: **A61F 2/02**

(86) Internationale Anmeldenummer:
**PCT/EP1998/000969**

(87) Internationale Veröffentlichungsnummer:
**WO 1998/036706 (27.08.1998 Gazette 1998/34)**

(54) **CHIRURGISCHES BESTECK**

SET OF SURGICAL INSTRUMENTS

JEU D'INSTRUMENTS CHIRURGICAUX

(84) Benannte Vertragsstaaten:
**AT BE CH DE DK ES FI FR GB GR IE IT LI LU MC NL PT SE**

(30) Priorität: **24.02.1997 DE 19708703**

(43) Veröffentlichungstag der Anmeldung:
**05.01.2000 Patentblatt 2000/01**

(73) Patentinhaber: **co.don Aktiengesellschaft**
**14513 Teltow (DE)**

(72) Erfinder:
• **ROTH, Klaus**
**D-72131 Ofterdingen (DE)**
• **SAILE, Hans-Jörg**
**D-72145 Hirrlingen (DE)**
• **SCHURR, Marc, O.**
**D-72074 Tübingen (DE)**

• **BUESS, Gerhard**
**D-72074 Tübingen (DE)**
• **JOSIMOVIC-ALASEVIC, Olivera**
**D-10717 Berlin (DE)**
• **FRITSCH, Karl-Gerd**
**D-14195 Berlin (DE)**

(74) Vertreter: **Ziebig, Marlene, Dr. Dipl.-Chem. et al**
**Patentanwälte**
**Gulde Hengelhaupt Ziebig & Schneider**
**Schützenstrasse 15-17**
**10117 Berlin (DE)**

(56) Entgegenhaltungen:
WO-A-96/27333          DE-U- 9 416 957
US-A- 3 017 887        US-A- 3 457 922
US-A- 5 171 244        US-A- 5 314 429
US-A- 5 330 468        US-A- 5 514 144

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die Erfindung betrifft ein chirurgisches Besteck zum Bearbeiten von Knorpel-, Knochen- oder Körpergewebeflächen, das ein chirurgisches Instrument 1 und eine Führungsvorrichtung 2 zum Führen dieses Instrumentes und/oder einen Gewebeapplikator 3 zum Einbringen von Gewebe an einen abzudeckenden Defekt umfaßt. Als chirurgisches Instrument, das mit Hilfe der erfindungsgemäßen Führungsvorrichtung 2 kreisförmig, elliptisch oder auch in anderer Form geführt werden kann, ist im Sinne der vorliegenden Erfindung ein Shaver, ein Skalpell, ein Bohrer, eine Kürette, eine Spritze oder auch eine Sonde zu verstehen. Je nach chirurgischer Fragestellung können jedoch auch andere Instrumente zum Schneiden, Fräsen, Stanzen, Nähen oder Anbringen von Bohrungen an Knorpel-, Knochen- oder Körpergewebe oder Einspritzen von Operationshilfsstoffen, wie z. B. Kleber, mit der erfindungsgemäßen Führungsvorrichtung in einer bestimmten Flächenform geführt werden.

Besonders vorteilhaft ist das erfindungsgemäße chirurgische Besteck zum mikroinvasiven Arbeiten an den verschiedensten Organen, so z.B. an der Bandscheibe, und ganz besonders zum arthroskopischen Arbeiten einsetzbar. So stellen z. B. Knorpeldefekte in der rekonstruktiven Gelenkchirurgie wegen der fehlenden Regenerationsfähigkeit des Knorpels ein großes Problem dar. Sie führen zu Störungen der Gelenkmechanik, zunehmendem Knorpelverschleiß in der Nachbarschaft und zu posttraumatischen Arthrosen.

Chirurgische Bestecke sind an sich bekannt. So wird in US-A-3,457,922 ein chirurgisches Besteck beschrieben, das ein chirurgisches Instrument, wie z.B. eine Bohrvorrichtung, und eine Führungseinrichtung zum orthogonalen Bearbeiten von Knochen- oder Körpergewebeflächen (Schädel oder Gehirn) aufweist.

In "Der Unfallchirurg", herausgegeben von E. Markgraf und K.E. Rehm, Springer Verlag, 60. Jahrestagung der Deutschen Gesellschaft für Unfallchirurgie e.V., 20.-23.11.1996 Berlin, Abstracts, Forum: Experimentelle Unfallchirurgie, Seite 283 wird ein Verfahren zur biotechnologischen Knorpelrekonstruktion mit kultivierten Chondrozyten und einem Kollagenschwamm beschrieben. Mit diesem Verfahren kann in-vitro autologes Knorpelgewebe in gewünschter Form und Größe hergestellt werden, indem die Knorpelzellen aus Kniescheiben frisch geschlachteter Kälber isoliert und auf einen Kollagenschwamm aufgetragen werden. Das Chondrozyten-Kollagenvlies-Konstrukt wird dann unter Zugabe von L-Ascorbinsäure, L-Glutamin, Antibiotika und fetalem Kälberserum kultiviert.

Anschließend wird dieses Konstrukt bei Nacktmäusen subkutan implantiert, und es entsteht neuer Knorpel in Form des verwendeten Kollagenschwammes. Es wird hyaliner Knorpel nachgewiesen und das für diesen Knorpel spezifische Kollagen Typ II.

Ein ähnliches Verfahren beschreibt die US-Patentschrift US 4.846.835. Diese Schrift offenbart, daß über dem Knorpeldefekt ein Flicken aus Knochenhaut (Periost) aufgebracht wird, nachdem das Chondrozyten-Kollagen-Material implantiert ist, um dieses mechanisch zu fixieren. Die US 4.846.835 beschreibt das Transplantationsverfahren an Kaninchen. Es werden zum Entnehmen des Periostgewebes und dessen Applikation keine Vorrichtun-gen beschrieben, die ein effizientes Arbeiten mit hoher Qualität bei gleichzeitiger Minimierung des Risikos für einen Patienten gewährleisten könnten. Es wird am offenen Knie gearbeitet, der Periostflicken mit einem Skalpell entnommen und auch die Läsion mit einem Skalpell präpariert.

Auch Matts Brittberg et al. beschreiben in "Clinical orthopaedics and related research No. 326, S. 270-283, 1996" diese Transplantationsmethode mit autologen Chondrozyten an neuseeländischen Kaninchen, wobei sie unter anderem auch die autologen Chondrozyten auf einem Kohlenstofffibervlies kultivieren und dieses Chondrozyten-Kohlenstofffibervlies-Konstrukt implantieren.

Diese Literaturstelle und auch zahlreiche andere Publikationen von Mats Brittberg et al. beschreiben jedoch ebenfalls ausschließlich Transplantations-verfahren am offenen Knie. Inzwischen wurde dieses Transplantationsverfahren am offenen Knie auch in der humanmedizinischen klinischen Praxis durchgeführt.

Allerdings bergen klassische Operationen am geöffneten Gelenk stets das Risiko einer Entzündung der Wunde in sich, der postoperative Schmerz ist beträchtlich, die Liegezeit in den Krankenhäusern länger, ebenso wie die Rehabilitationszeit.

Aufgabe der vorliegenden Erfindung ist es, ein chirurgisches Besteck zu entwickeln, das eine akkurate Führung des chirurgischen Instrumentes je nach der zu bearbeitenden Fläche erlaubt, mit dem sowohl am offenen Defekt als auch mikroinvasiv gearbeitet werden kann und das insbesondere auch für arthroskopisches Arbeiten geeignet ist.

Insbesondere ist es die Aufgabe der Erfindung, ein effizientes Transplantationsverfahren zur biotechnologischen Knorpelrekonstruktion am Menschen zu entwickeln, das die in-vitro-Kultivierung von autologen Knorpelzellen und deren Transplantation in den Knorpeldefekt sowie das Verschließen des Defektes mittels Periostgewebe ausnutzt, aber kein Arbeiten am offenen Gelenk notwendig macht.

Die Aufgabe der Erfindung wird durch ein chirurgisches Besteck umfassend ein chirurgisches Instrument (1) und eine Führungsvorrichtung (2) zum orthogonalen oder horizontalen Bearbeiten von Knorpel-, Knochen- oder Körpergewebeflächen gemäß der Ansprüche gelöst. Die Führungsvorrichtung beeinhaltet ein körpernahes und ein körperfernes Führungselement, die eine reproduzierbare Bewegung des chirurgischen Instrumentes (1) durch einen invarianten Punkt P erlauben, wobei durch Verändern des Abstandes zwischen den beiden Führungselementen die Größe der zu bearbeitenden Fläche (vorzugsweise ein Kreis- oder eine Ellipse) variiert werden kann.

Dabei bestimmt die Relation des Abstandes zwischen den beiden Führungeselementen und des Abstandes des invarianten Punktes zur zu bearbeitenden Gewebefläche unter gleichzeitiger Berücksichtigung des Winkels zwischen den gedachten Geraden a und b die Größe der zu bearbeitenden Gewebefläche, wobei Gerade a durch den invarianten Punkt und zentral durch die von den Führungselementen gebildete geometrische Figur verläuft, während Gerade b durch den invarianten Punkt und tangential zum entferntesten Punkt durch die von den Führungselementen gebildete geometrische Figur verläuft.

Bevorzugte Ausführungsformen der Führungsvorrichtung (2) sind in den Figuren 1 bis 3 und 5 bis 8 dargestellt.

Es zeigen

Fig. 1: Schnittdarstellung einer Ausführungs-variante der Führungsvorrichtung 2 zum senkrechten Aufsetzen auf die zu bearbeitende Fläche

Fig. 2: Darstellung einer weiteren Ausführungsvariante der Führungsvorrichtung 2 zum senkrechten Aufsetzen auf die zu bearbeitende Fläche

Fig. 3: Darstellung einer Ausführungsvariante der Führungsvorrichtung 2 zum tangentialen Aufsetzen

Fig. 5,6,7: Darstellungen von erfindungsgemäßen Ausführungsvarianten des Gewebeapplikators

Fig. 8: Detaillierte Schnittdarstellung der Führungsvorrichtung 2 gemäß Fig. 3

Das chirurgische Besteck besteht erfindungsgemäß aus dem chirurgischen Instrument 1 und einer Führungseinheit 2, in der das chirurgische Instrument 1 so geführt wird, daß je nach Größe des Defektes unterschiedlich große Flächen bearbeitet werden können. Gegebenenfalls umfaßt das Besteck einen Gewebeapplikator 3 zum Einbringen von Gewebe an einen abzudeckenden Defekt.

Während die Führungsvorrichtung gemäß der Ansprüche 1 - 4 zum senkrechten Aufsetzen auf eine z. B. mikroinvasiv zu schneidende, fräsende oder mit Bohrungen zu versehende Knorpel-, Knochen- oder Körpergewebefläche entwickelt ist, kann mit der Führungsvorrichtung gemäß der Ansprüche 5 und 6 tangential gearbeitet werden. So stellt sich z. B. in der Arthroskopie vergleichsweise oft das Problem, daß nicht in senkrechter Richtung an der betroffenen Partie am Knie gearbeitet werden kann, sondern die Instrumente seitlich eingeführt werden müssen und tangential gearbeitet werden muß. Insbesondere ist dies bei Defekten auf der Rückseite der Kniescheibe der Fall.

Gemäß der Erfindung kann das chirurgische Instrument 1 auch eine Spritze sein, die in der Führungsvorrichtung 2 geführt wird, so daß über eine lange Kanüle notwendige Hilfsstoffe für die Operation, zum Beispiel Fibrinkleber, gezielt vor Ort auf die Umrandung einer mit der Führungsvorrichtung 2 einstellbaren, und möglicherweise vorher mit einem Shaver ausgefrästen und nun zu beklebenden Fläche aufgebracht werden können.

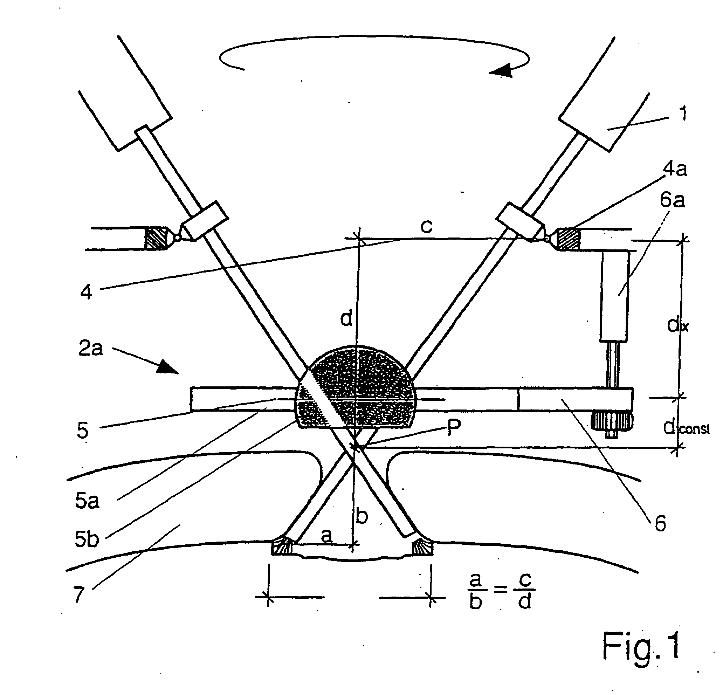

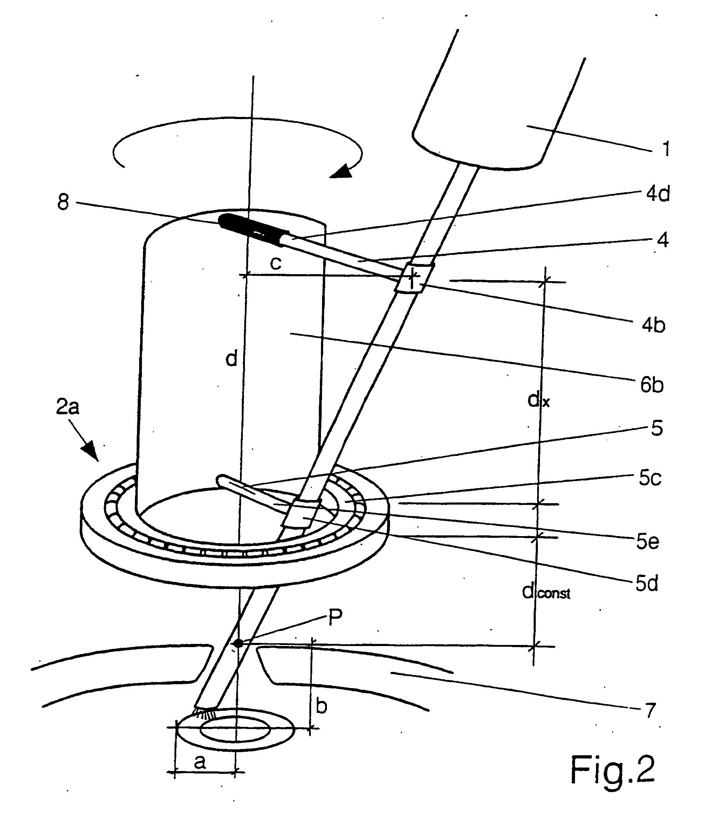

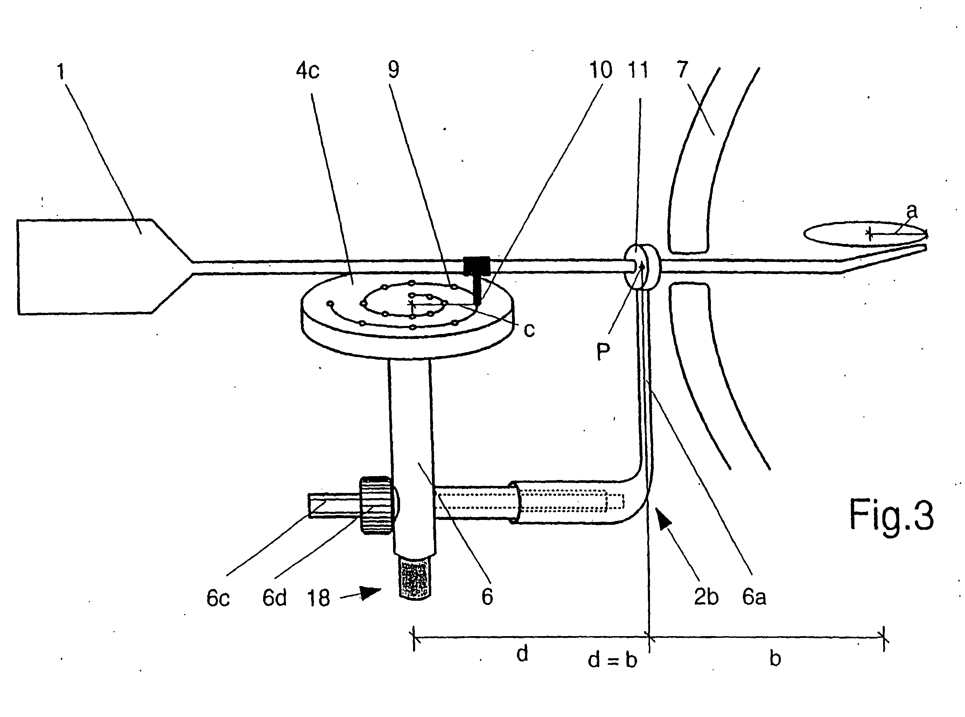

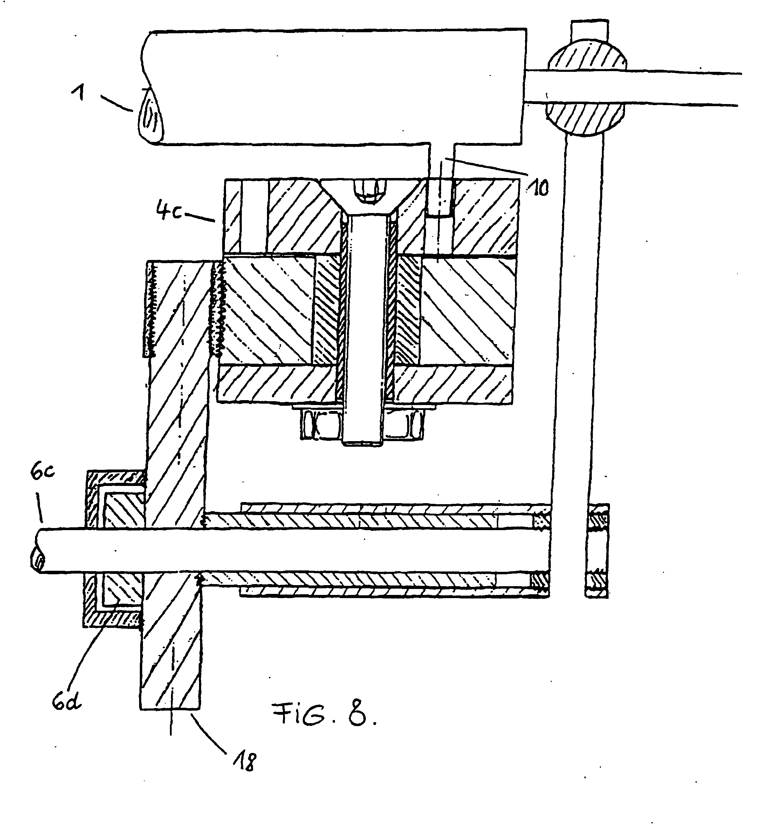

Bevorzugte Ausführungsvarianten zum orthogonalen Bearbeiten der Gewebeflächen werden in den Figuren 1 und 2 gezeigt. Figuren 3 und 8 zeigen bevorzugte Ausführungsformen der Führungsvorrichtung zum horizontalen Bearbeiten von Knorpel-, Knochen oder Körpergewebeflächen.

Eine erste bevorzugte Ausführungsvariante der Führungseinheit 2 zum orthogonalen Bearbeiten ist in Figur 1 im Schnitt dargestellt und in Anspruch 2 beschrieben.

Das Prinzip der Führung des chirurgischen Instrumentes 1 bei dieser Variante beruht darauf, daß zwei Führungselemente verschiedener Durchmesser 4a und 5a in einem verstellbaren Abstand d konzentrisch zueinander gelagert sind. Der obere, größer gestaltete Führungsring 4a dient als Aufnahme für das chirurgische Instrument 1. Der Radius c des Ringes ist konstant. Im unteren Führungsring 5a ist eine horizontal drehbare Kugel 5b gelagert, die eine exzentrische Bohrung hat. Durch diese Bohrung wird das chirurgische Instrument 1 hindurchgeführt. Durch die Änderung des Abstandes d der beiden Führungsringe zueinander kann so der Anstellwinkel des chirurgischen Instrumentes 1 variiert werden.

Die Bohrung in der Kugel 5b des unteren Führungsringes 5a muß exzentrisch sein, damit der invariante Punkt P unterhalb des unteren Führungsringes 5a zum Liegen kommt. Der invariante Punkt sollte der Durchtrittspunkt durch die Haut sein und in der Mitte der Hautschicht liegen. So ist es möglich, daß das chirurgische Instrument entlang des oberen Führungsringes 4a geführt werden kann, und der Durchtrittspunkt durch die Haut immer an der gleichen Stelle bleibt. Durch die verschiedenen Größen des oberen und des unteren Führungsringes 4a, 5a beeinflußt der Abstand d zwischen beiden Ringen die Größe der zu bearbeitenden Fläche gemäß der Gleichung a:b = c:d, so daß durch Verändern des Abstandes d der beiden Führungsringe zueinander der Radius a der zu bearbeitenden Fläche eingestellt werden kann. Dabei entspricht die Strecke b dem Abstand zwischen dem invarianten Punkt P und dem Mittelpunkt der zu bearbeitenden Fläche. Dieser Abstand zwischen dem unteren Führungsring 5a und dem invarianten Punkt P ist konstant.

Eine zweite bevorzugte Ausführungsvariante der Führungsvorrichtung 2 zum orthogonalen Arbeiten ist aus Figur 2 ersichtlich und in Anspruch 3 beschrieben.

Bei dieser Ausführungsvariante ist in einem inneren Ring 5c eines Lagers, vorzugsweise eines Kugellagers, eine Halbschale 6b eines Rohres senkrecht zur Ringebene drehbar angeordnet. Der Innendurchmesser dieses Rohres ist idealerweise geringfügig größer als der Außenumfang des chirurgischen Instrumentes 1. An der Aufnahmestelle der Halbschale im Lager befindet sich eine im Durchmesser des Lagers verschiebbare untere Aufnahmebuchse 5d für das chirurgische Instrument 1. Diese Aufnahmebuchse 5d stellt die untere Führungsebene dar. Der Abstand der Aufnahmebuchse 5d vom Mittelpunkt des Durchmessers durch das Kugellager beeinflußt die Lage des invarianten Punktes P unterhalb des Lagers. Am oberen Ende der Halbschale 6b befindet sich ein auslenkbares Befestigungselement 8 für das chirurgische Instrument 1. An diesem Befestigungselement ist die obere Aufnahmebuchse 4b über ein Verbindungselement 4d befestigt, so daß bei Rotation der Halbschale eine kreisförmige obere Führungsebene 4 entsteht. Um die zu bearbeitende kreisförmige Fläche mit dem Radius a im Patienten zu vergrößern, muß das Befestigungselement 8 ausgelenkt werden. In dieser Ausführungsvariante stellt der Radius c den senkrechten Abstand des Mittelpunktes der oberen Aufnahmebuchse 4b zur Mittellinie der Halbschale 6b dar.

Eine Führungsvorrichtung zum tangentialen Aufsetzen auf eine zu schneidende, fräsende, mit Bohrungen zu versehende oder zu umnähende Fläche ist in Anspruch 5 beschrieben und in den Abbildungen 3 und 8 gezeigt. Basierend auf der ersten bevorzugten Ausführungsvariante der Führungsvorrichtung 2 zum senkrechten Aufsetzen wird im vorliegenden Fall die obere Führungsebene 4 um 90° gedreht, so daß die obere Führungsebene, die im vorliegenden Fall die Form einer Scheibe 4c hat, mit einer Halterung 6 fest verbunden ist und mit dieser eine Ebene bildet. Das chirurgische Instrument 1 wird über einen Führungsbolzen 10 in eine der auf der Scheibe schneckenförmig angebrachten Bohrungen 9 gesteckt, so daß durch Drehen der Scheibe eine Drehbewegung des chirurgischen Instrumentes 1 erreicht wird und damit eine tangentiale Bearbeitung der Läsion möglich ist. Bei dieser Variante wird also das chirurgische Instrument 1 indirekt geführt. Ist die zu bearbeitende Fläche kreisförmig, wird der Radius a der zu bearbeitenden Fläche durch den jeweiligen Radius c vom Mittelpunkt der Scheibe zur Bohrung, in die das chirurgische Instrument 1 eingesteckt wurde, bestimmt. Das bedeutet auch, daß die Strecken b und d gleich lang sein können. Alternativ kann die Führungsvorrichtung auch so angesetzt werden, daß keine kreisförmige, sondern eine elliptische Fläche bearbeitet wird. Über eine Gewindestange 6c läßt sich mittels Rändelmutter 6d in einer besonders bevorzugten Ausführungsform der Abstand d verstellen (vgl. Fig. 3 und 8). Wie unschwer zu erkennen ist, ist es in der vorliegenden Variante vorteilhaft, daß der Kopf des chirurgischen Instrumentes 1 gebogen ist. Mit diesen leicht gekröpften Instrumenten ist es möglich, jeden Punkt zu erreichen, unter anderem auch die Rückseite der Kniescheibe.

In allen Ausführungsvarianten der erfindungsgemäßen Führungsvorrichtung ist es möglich, eines der Führungselemente 4 oder 5 mit einer Graduierung zu versehen, so daß z. B. Bohrlöcher, die mit dem chirurgischen Instrument 1 im Knorpel oder Knochen gesetzt wurden, durch die Graduierung bestimmt und möglicherweise auf ein Stück Gewebe, das auf diesem Defekt zum Abdecken aufgebracht werden soll, übertragen werden können. So kann die Position möglicher einzubringender Knochenstifte (Pins) schon im Gewebe passend vorgeprägt werden.

Es sind selbstverständlich weitere Ausführungsformen des erfindungsgemäßen Prinzips denkbar, die der Fachmann, ohne erfinderisch tätig zu werden, umsetzen kann. So kann die Führungsvorrichtung der Figur 3 so gestaltet werden, daß bei Unterbrechung des gleichmäßigen Führens des chirurgischen Instrumentes 1 durch Vor- und Zurückfahren nicht nur runde Formen, sondern auch Kerben oder Überstände in der Form möglich und durch die Graduierung bestimmbar und übertragbar (z. B. auf Gewebe) werden. Dies kann z. B. in der Ausführungsvariante der Fig. 3 erreicht werden, indem der Führungsbolzen 10 am Schaft des chirurgischen Instrumentes 1 transversal verschiebbar angebracht ist.

Es lassen sich mit dem erfindungsgemäßen chirurgischen Instrument 1 und der dazugehörigen Führungsvorrichtung 2 nicht nur Übertragungen der Fläche eines Defektes nach außen, sondern auch dreidimensionale Übertragungen bewerkstelligen, indem z. B. an einen Knochen- oder Knorpeldefekt mittels einer doppellumigen Spritze 1 zwei Komponenten gebracht werden, die vor Ort vernetzen (z.B. ein Biopolymer und ein Vernetzungsmittel). Somit kann der Defekt modelliert werden.

Selbstverständlich ist das erfindungsgemäße chirurgische Instrument 1 mit seiner Führungsvorrichtung 2 auch elektronisch steuerbar oder kann energetisch angetrieben werden. Auch sind mittels der Führungsvorrichtung 2 und einer Sonde als chirurgischem Instrument 1 Defekte abtastbar und nach außen übertragbar.

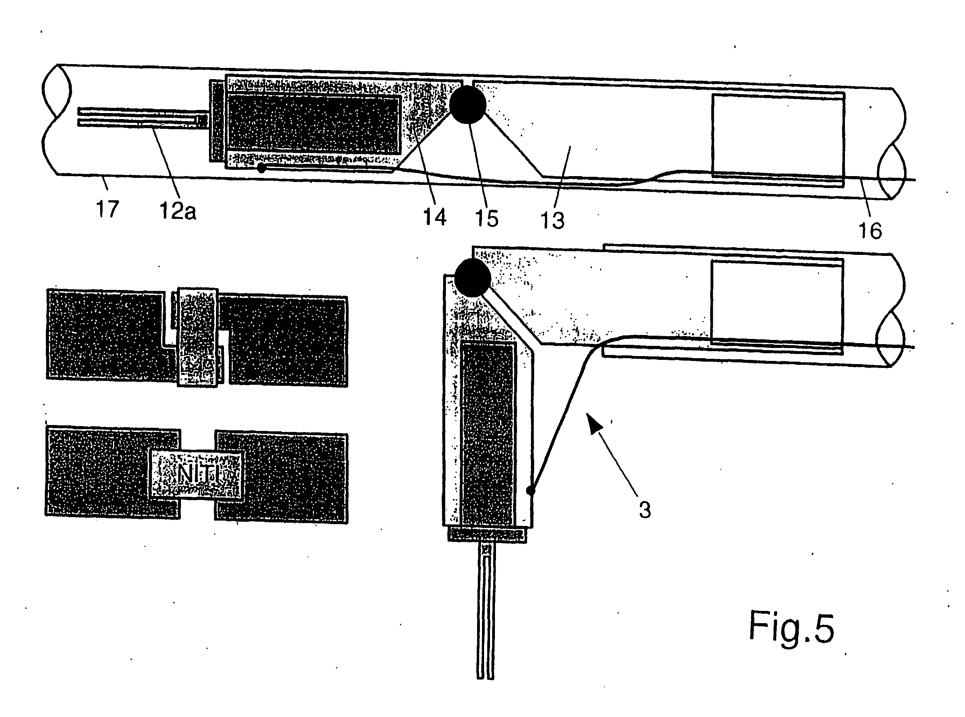

Ein möglicher weiterer Bestandteil des erfindungsgemäßen chirurgischen Besteckes ist ein Gewebeapplikator 3 zum Einbringen von Gewebe an einen abzudeckenden Defekt. Als Gewebe wird im Sinne der Erfindung jegliches vom Körper resorbierbares Gewebe, aber auch Periost oder andere körpereigene Gewebe verstanden. Gemäß der Erfindung besteht der Gewebeapplikator 3 aus einer Halterung 12 für das Gewebe, die drehbar und/oder auslenkbar an einem Rohrschaft 13 gelagert ist. Eine bevorzugte Ausführungsvariante eines solchen Gewebeapplikators 3 ist in Figur 5 dargestellt. Dieser dargestellte Gewebeapplikator 3 besitzt eine gabelförmige Halterung 12a, die der Länge des Gewebeflickens angepaßt werden kann und in einem gegenüber dem Rohrschaft 13 gegebenenfalls abwinkelbaren Aufnahmeelement 14 drehbar gelagert ist. Zur Abwinkelung ist im vorliegenden Fall eine Gelenkverbindung 15 vorgesehen, aber auch eine superelastische Verbindung zwischen Rohrschaft 13 und Aufnahmeelement 14 ist möglich. In der praktischen Anwendung wird der Gewebeapplikator in einem Überrohr 17 durch eine sogenannte Trokarhülse in den Körper eingeführt. Über einen Steuerdraht 16 wird die Abwinkelung des Aufnahmeelementes gesteuert.

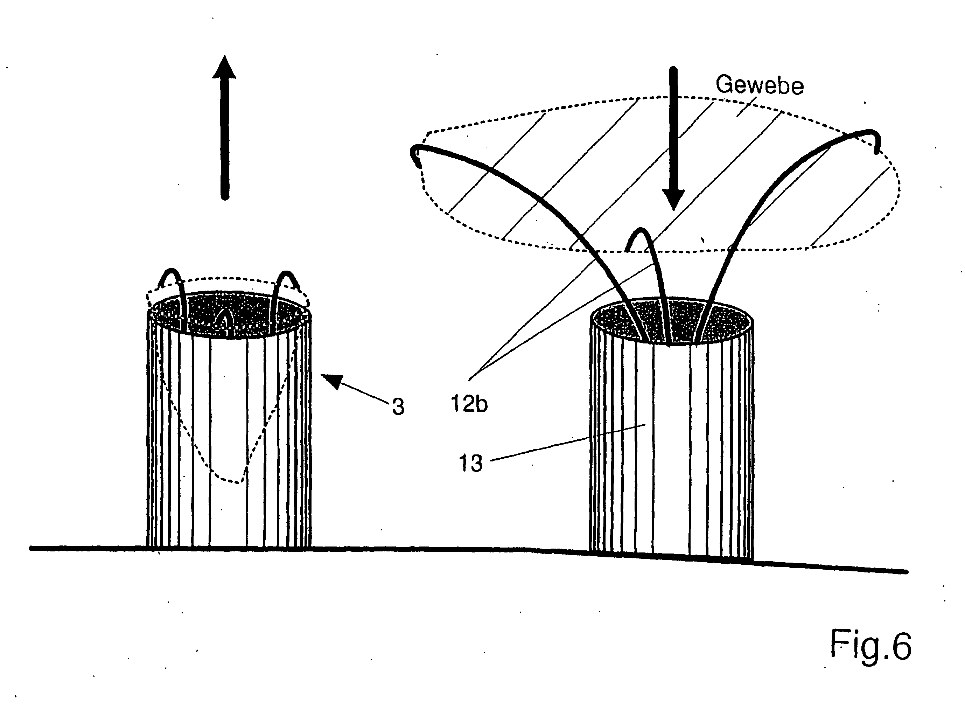

Eine weitere mögliche Ausführungsvariante des erfindungsgemäßen Gewebeapplikators 3 ist in Anspruch 10 beschrieben und in Figur 6 dargestellt. Bei dieser Ausführungsform besteht die Halterung 12b aus Haken, die im Rohrschaft 13 auslenkbar angeordnet sind. Die Haken müssen hochelastisch sein und bestehen selbstverständlich aus einem medizinisch zugelassenen Material, z. B. einer Nickel-Titan-Legierung. Der Gewebeflicken wird an den Haken 12b eingehängt und in den Rohrschaft 13 gezogen oder mittels eines Hilfsmittels eingeschoben. Beim Wiederausschieben der Haken 12b spannen diese aufgrund ihrer Elastizität das Gewebe wie einen Regenschirm auf.

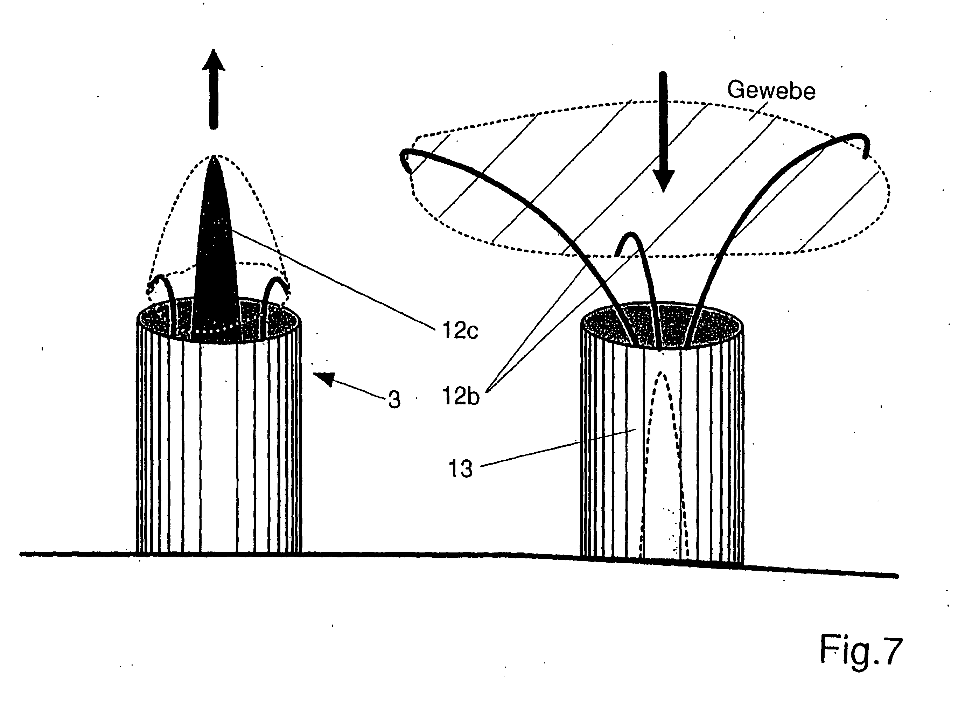

In einer weiteren Ausführungsvariante des erfindungsgemäßen Gewebeapplikators 3 besitzt dieser gemäß Fig. 7 zusätzlich einen auslenkbaren Dorn 12c. Dieser kann aus einem medizinisch zugelassenen Metall oder Kunststoff bestehen. Diese Variante gewährleistet, daß das Gewebe, insbesondere Periost, nicht in den Rohrschaft 13 eingebracht werden muß, da dies aufgrund des Größenmißverhältnisses schwierig sein kann. Durch die erfindungsgemäße Variante der Fig. 7 ist das Gewebe an den Haken 12b eingehängt und über den ausgefahrenen Dorn 12c gespannt. Beim Zurückziehen des Dorns 12c mit gleichzeitiger Ausfahrung der Haken 12b wird das Gewebe aufgespannt und kann nun auf den Defekt aufgebracht werden.

Der erfindungsgemäße Gewebeapplikator 3 kann prinzipiell bei jedem Einbringen von Gewebe in den Körper von Nutzen sein, so z. B. bei der Knorpelrekonstruktion am Knie zum Einbringen des Periostes oder beim Einbringen eines Netzes zur Reparatur eines Leistenbruches.

Aus den Ausführungen wird deutlich, daß die erfindungsgemäße Führungsvorrichtung 2 zusammen mit einem Shaver als chirurgischem Instrument 1 z. B. bei der Behandlung von Knorpeldefekten sowohl zum Ausschneiden des gesunden Knorpels, der in-vitro kultiviert werden soll, zum Anbringen einer Nut um den Knorpeldefekt und zum Anschneiden des Periostgewebes, das den Knorpeldefekt abdecken soll, dienen kann.

Die Erfindung betrifft deshalb neben dem chirurgischen Besteck auch ein Transplantationsverfahren zur biotechnologischen Knorpelrekonstruktion, das die in-vitro-Kultivierung von autologen Knorpelzellen und deren Transplantation in den ausgeschälten und mit einer Ringnut präparierten Knorpeldefekt sowie das Verschließen des Defektes mittels Periostgewebe ausnutzt. Das Transplantationsverfahren wird unter Anwendung des erfindungsgemäßen chirurgischen Besteckes gemäß der Ansprüche 1 bis 12 durchgeführt. So kann die Führungsvorrichtung 2 und ihre bevorzugten Ausführungsvarianten gemäß den Figuren 1 und 2 und einem Skalpell als chirurgischem Instrument 1 bei der kreisrunden Entfernung vom Periost am Schienbein angewandt werden. Ebenso dienen diese Vorrichtungen zum Anbringen einer Ringnut um Defekte im Kniebereich, wenn diese direkt von vorn zugänglich sind. Daneben kann die Führungsvorrichtung 2 mit einer Spritze als chirurgischem Instrument 1 zum Ankleben des Periostflickens an der Ringnut angewandt werden. Sind die Defekte im Kniebereich nur seitlich zugänglich, so muß die Führungsvorrichtung 2 gemäß der Figur 3 eingesetzt werden.

In einer bevorzugten Ausführungsvariante wird das erfindungsgemäße Transplantationsverfahren mit dem erfindungsgemäßen Besteck zur Behebung eines von vorn zugänglichen Knorpeldefektes wie folgt durchgeführt:

Ein Knorpeldefekt am Knie wird bis auf die operativ nötige Tiefe ausgearbeitet. Da der Knorpeldefekt von vorn zugänglich ist, wird die Führungsvorrichtung 2 gemäß Fig. 1 über bekannte Haltesysteme angebracht und entsprechend eingestellt. Die Größe des Defektes gibt den zu wählenden Abstand zwischen oberem und unterem Führungsring 4a, 5a vor. Dadurch kann die Größe des Kreises die der Shaver im Knie beschreibt, bestimmt werden. Die nun zu bearbeitende Fläche sollte idealerweise etwas größer als der größte Durchmesser des Defektes gewählt werden, so daß mit dem Shaver eine kreisrunde Stufe um den Defekt gefräst werden kann.

In einem zweiten Arbeitsschritt wird nun mit Hilfe der Führungsvorrichtung 2 am Schienbein des Patienten Knochenhaut (Periost) angeschnitten. Durch diesen Schnitt wird die Größe des Periostflickens vorgegeben. Auf Grund des Abstandes zwischen unterem und oberem Führungsring, der im Arbeitsschritt 1 bereits bestimmt worden ist, hat der Periostflicken die richtige Größe und kann anschließend bei der ReTransplantation am Knie sauber in die ausgearbeitete Stufe eingepasst werden. Das Periost wird nun mit üblichen artroskopischen Instrumenten vom Knochen gelöst und geborgen. Bis zur Transplantation muß es steril aufbewahrt werden.

In einem dritten Arbeitsschritt wird das Periost z. B. auf den erfindungsgemäßen Gewebeapplikator gemäß Fig. 5 aufgewickelt und in das Knie eingebracht. Sollte es sich im Laufe der Untersuchung herausstellen, daß es notwendig ist, für die Knochenstifte Bohrungen vorzusehen, damit diese auch in die Knorpelmasse einfach eingebracht werden können, sollten am Knie diese Bohrungen mit einem Bohrer als chirurgisches Instrument 1 unter Zuhilfenahme der Führungsvorrichtung 2 angebracht werden. Für diesen Zweck besitzt der obere Führungsring 4a eine Gradeinteilung, die ein Übertragen der Bohrlöcher aus dem Knie auf den Periostflicken ermöglicht. Der Periostflicken wird also unter Zuhilfenahme der erfindungsgemäßen Führungsvorrichtung 2 mit Bohrer oder Kürette als chirurgischem Instrument 1 perforiert. Durch diese Verfahrensweise vereinfacht sich das Wiederfinden der Bohrlöcher durch das Periost hindurch bei der Applikation im Knie wesentlich.

Nun wird die Knochenhaut mit einem ersten Knochenstift (Pin) durch die Perforation im Knorpel des Knies befestigt. Durch seitliches Bewegen des Gewebeapplikators 3 wird der Periostflicken danach soweit abgewickelt, bis ein zweiter Pin zu setzten ist. Für weitere Pins kann dann das Periost noch weiter abgewickelt werden, bis das Periost mit der nötigen Anzahl Pins befestigt worden ist. Die nötige Anzahl der Pins ergibt sich aus der Größe des jeweils zu behandelnden Defektes.

In einem vierten Arbeitsschritt wird zum Abdichten des Periostflickens zum gesunden Knorpelgewebe hin eine doppellumige Spritze, die mit Fibrinkleber gefüllt ist, in die Führungseinheit 2 eingespannt. Über eine lange doppellumige Kanüle wird dann direkt das Fibrin gespritzt. So kann mit einer sehr geringen Menge an Fibrin der Periostflicken zum Knorpel hin abgedichtet werden, da das Fibrin genau vor Ort durch die Einstellung der Führungseinheit 2 plaziert wird.

Es hat sich gezeigt, daß ein solches erfindungsgemäßes "Ankleben" des Periostes an die gefräste Nut so haltbar ist, daß gegebenenfalls auch auf das Anbringen von Knochenstiften völlig verzichtet werden kann.

Im fünften und letzten Arbeitsschritt werden nun die in vitro kultivierten autologen Chondrozyten unter das Periost injiziert und die Operationswunde geschlossen.

**Bezugszeichenliste**

1 Chirurgisches Instrument

2 Führungsvorrichtung für das chirurgische Instrument

3 Gewebeapplikator

4 oberes Führungselement

4a oberer Ring

4b obere Aufnahmebuchse

4c drehbare Scheibe

4d Verbindungselement

5 unteres Führungselement

5a unterer Ring

5b Kugel oder Halbkugel mit exzentrischer Bohrung

5c innerer Ring eines Lagers

5d untere Aufnahmebuchse

5e Verbindungselement

6 Halterung

6a Verbindungselement

6b Halbschale

6c Gewindestange

6d Rändelmutter

7 Haut

8 längenverstellbares Befestigungselement

9 Bohrungen

10 Führungsbolzen am chirurgischen Instrument

10a längensverstellbarer Führungsbolzen

11 Führungsbuchse

12 Halterung für Gewebe

12a gabelförmige Halterung

12b Halterung bestehend aus Haken

12c Dorn

13 Rohrschaft des Gewebeapplikators

14 Aufnahmeelement

15 Gelenkverbindung

16 Steuerdraht zur Abwinkelung

17 Überrohr

18 Tischhalterung

a Radius der zu bearbeitenden Fläche

b Abstand zwischen invariantem Punkt P und der zu bearbeitenden Fläche

c Radius der oberen Führungsebene

d Abstand zwischen der oberen und der unteren Führungsebene bzw. zwischen der oberen Führungsebene und der unteren Führungsbuchse (11)

y Kurvenausgleichsstrecke (Strecke sinus a vom Winkel α)

P invarianter Punkt

**Patentansprüche**

1. Chirurgisches Besteck umfassend ein chirurgisches Instrument (1) und eine Führungsvorrichtung (2) zum orthogonalen Bearbeiten von Knorpel-, Knochen- oder Körpergewebeflächen, **dadurch gekennzeichnet,** **daß** in der Führungsvorrichtung (2) ein Führungselement (4) zum kreisförmigen Führen des chirurgischen Instrumentes (1) und ein horizontal drehbares Führungselement (5) vorhanden und beide Führungselemente (4,5) über eine Halterung (6) verbunden sind, wobei das kreisförmig drehbare Führungselement (4) so angeordnet ist, daß der Radius c der beim kreisförmigen Führen des Instrumentes (1) entstehenden Führungsebene größer ist als der Radius der mit dem horizontal drehbaren Führungselement entstehenden kreisförmigen Führungsebene und das chirurgische Instrument (1) exzentrisch durch diese Führungsebene so hindurchgeführt ist, daß es unterhalb des horizontal drehbaren Führungselementes (5) durch einen invarianten Punkt (P) tritt und durch die Veränderung des Abstandes d der beiden Führungselemente (4,5) zueinander der Radius a der mit dem chirurgischen Instrument (1) zu bearbeitenden Fläche gemäß der Gleichung

$$a : b = c : d$$

einstellbar ist, wobei b den Abstand zwischen dem invarianten Punkt (P) und dem Mittelpunkt der zu bearbeitenden Fläche darstellt.

2. Chirurgisches Besteck nach Anspruch 1, **dadurch gekennzeichnet,** **daß** in der Führungsvorrichtung (2) das kreisförmig drehbare Führungselement (4) ein Ring (4a) ist, das horizontal drehbare Führungselement (5) aus einem Ring (5a) und einer darin gelagerten Kugel oder Halbkugel (5b) mit exzentrischer Bohrung besteht, durch die das chirurgische Instrument (1) hindurchgeführt wird, die beiden Ringe (4a,5a) über ein Verbindungselement (6a) miteinander verbunden sind und der untere Ring (5a) über die Halterung (6) am Verbindungselement (6a) höhenverstellbar angebracht ist.

3. Chirurgisches Besteck nach Anspruch 1, **dadurch gekennzeichnet,** **daß** in der Führungsvorrichtung (2) das horizontal drehbare Führungselement (5) der innere Ring (5c) eines Lagers ist, in welchem senkrecht zur Ringebene (5c) eine Halbschale (6b) drehbar angeordnet ist, an deren im Lager befindlichen unterem Ende über ein Verbindungselement (5e) eine Aufnahmebuchse (5d) für das chirurgische Instrument (1) so befestigt ist, daß das chirurgische Instrument (1) exzentrisch durch den inneren Ring (5c) des Lagers hindurchgeführt wird, und an deren Ende an einem längenverstellbaren Befestigungselement (8) über ein Verbindungselement (4d) eine Aufnahmebuchse (4b) für das chirurgische Instrument (1) befestigt ist, so daß der Radius c der beim kreisförmigen Führen des Instrumentes (1) entstehenden Führungsebene der senkrechte Abstand des Mittelpunktes der Aufnahmebuchse (4b) zur Mittellinie der Halbschale (6b) ist.

4. Chirurgisches Besteck nach einem der Ansprüche 1 bis 3, **dadurch gekennzeichnet,** **daß** eines der Führungselemente (4) oder (5) eine Graduierung aufweist.

5. Chirurgisches Besteck umfassend ein chirurgisches Instrument (1) und eine Führungsvorrichtung (2) zum Bearbeiten von Knorpel-, Knochen- oder Körpergewebeflächen in einer Ebene mit den zu bearbeitenden Flächen, **dadurch gekennzeichnet,** **daß** es ein chirurgisches Instrument (1) mit einem gebogenen Kopf und einem Führungsbolzen (10) am Schaft oder am Griff des Instrumentes beinhaltet, und die Führungsvorrichtung (2) ein kreisförmiges Führungselement (4) in Form einer drehbaren Scheibe (4c) mit exzentrischen, schneckenförmig angeordneten Bohrungen (9) zum wahlweisen Einstecken eines am Schaft oder am Griff des chirurgischen Instrumentes vorhandenen Führungsbolzens (10) hat, wobei die Entfernung der jeweiligen Bohrung (9), in die der Führungsbolzen (10) eingesteckt wird, zum Mittelpunkt der Scheibe (4c) als Radius c bezeichnet wird, und diese Scheibe (4c) mit der zu behandelnden Fläche in einer Ebene liegt und mit einer Halterung (6) fest verbunden ist, wobei am unteren Ende der Halterung (6) über ein senkrechtes Verbindungselement (6a) eine Führungsbuchse (11) für das chirurgische Instrument (1) befestigt ist, deren Mittelpunkt gleichzeitig einen invarianten Punkt (P) darstellt, und die Größe der zu bearbeitenden Fläche bestimmt wird durch die Wahl des Radiusses c auf der Scheibe (4c) und einem einstellbaren Abstand d zwischen dem Mittelpunkt der Scheibe (4c) und dem Mittelpunkt der Führungsbuchse (11).

6. Chirurgisches Besteck nach Anspruch 5,
   **dadurch gekennzeichnet,**
   **daß** das Führungselement (4) in der Führungsvorrichtung (2) eine Graduierung aufweist.

7. Chirurgisches Besteck nach einem der Ansprüche 1 bis 6,
   **dadurch gekennzeichnet,**
   **daß** es zusätzlich einen Gewebeapplikator (3) zum Einbringen von Gewebe an einen abzudeckenden Defekt umfaßt, der aus einer Halterung (12) für das Gewebe und einem Rohrschaft (13) besteht, wobei die Halterung (12) im Rohrschaft (13) gegebenenfalls drehbar und/oder auslenkbar gelagert ist.

8. Chirurgisches Besteck nach Anspruch 7,
   **dadurch gekennzeichnet,**
   **daß** der Gewebeapplikator (3) eine gabelförmige Halterung (12a) besitzt, die in einem, gegebenenfalls gegenüber dem Rohrschaft (13) abwinkelbaren, Aufnahmeelement (14) drehbar gelagert ist.

9. Chirurgisches Besteck nach Anspruch 8,
   **dadurch gekennzeichnet,**
   **daß** zur Abwinkelung zwischen Rohrschaft (13) und Aufnahmeelement (14) eine Gelenkverbindung (15) vorhanden ist.

10. Chirurgisches Besteck nach Anspruch 7,
    **dadurch gekennzeichnet,**
    **daß** der Gewebeapplikator (3) eine Halterung (12b) aus Haken besitzt, die im Rohrschaft (13) auslenkbar angeordnet sind.

11. Chirurgisches Besteck nach Anspruch 10,
    **dadurch gekennzeichnet,**
    **daß** der Gewebeapplikator zusätzlich einen auslenkbaren Dorn (12c) zum Aufspannen des Gewebes besitzt.

**Claims**

1. A surgical set of instruments, comprising a surgical instrument (1) and a guide device (2) for the orthogonal treatment of cartilage, bone or body tissue surfaces,
   **characterized in that**
   the guide device (2) has a guide. element (4) for circular guiding of the surgical instrument (1) and a horizontally rotatable guide element (5), and both guide elements (4, 5) are connected via a holder (6), wherein the guide element (4) rotatable in a circular fashion is arranged in such a way that the radius c of the guide plane generated in circular guiding of instrument (1) is greater than the radius of the circular guide plane generated with the horizontally rotatable guide element, and the surgical instrument (1) passes eccentrically through this guide plane in a way so as to pass through an invariant point (P) below the horizontally rotatable guide element (5), and the radius a of the area to be treated with the surgical instrument (1) can be adjusted by varying the distance d of the two guide elements (4, 5) relative to each other according to equation

$$a : b = c : d$$

where b is the distance between the invariant point (P) and the center of the area to be treated.

2. The surgical set of instruments according to claim 1,
   **characterized in that**
   the circularly rotatable guide element (4) in guide device (2) is a ring (4a), the horizontally rotatable guide element (5) consists of a ring (5a) and a ball or hemisphere (5b) which is arranged in a bearing therein and has an eccentric bore where the surgical instrument (1) is passed through, the two rings (4a, 5a) are joined to each other by a joint element (6a), and the lower ring (5a) is arranged vertically adjustable on joint element (6a) via holder (6).

3. The surgical set of instruments according to claim 1,
   **characterized in that**
   the horizontally rotatable guide element (5) in guide device (2) is the inner ring (5c) of a bearing wherein a half-shell (6b) is rotatably arranged vertically to the ring plane (5c), at the bottom of which situated in the bearing an accommodation sleeve (5d) for the surgical instrument (1) is affixed via a joint element (5e) in such a way that the surgical instrument (1) passes eccentrically through the inner ring (5c) of the bearing, and at the top of which an accommodation sleeve (4b) for the surgical instrument (1) is affixed to a longitudinally adjustable securing element (8) via a joint element (4d), so that the radius c of the guide plane generated in circular guiding of the instrument (1) is equal to the vertical distance from the center of the accommodation sleeve (4b) to the center line of the half-shell (6b).

4. The surgical set of instruments according to any of claims 1 to 3,
   **characterized in that**
   one of the guide elements (4) or (5) has a graduation.

5. A surgical set of instruments, comprising a surgical instrument (1) and a guide device (2) for the treatment of cartilage, bone or body tissue surfaces in the same plane as the areas to be treated, **characterized in that** the set includes a surgical instrument (1) having a bent head and a guide pin (10) on its shank or grip, and the guide device (2) has a circular guide element (4) in the form of a rotatable disk (4c) having eccentric, helically arranged bores (9) for optional insertion of a guide pin (10) present on the shank or grip of the surgical instrument, wherein the distance from the respective bore (9), into which the guide pin (10) is inserted, to the center of the disk (4c) is referred to as radius c, and said disk (4c) is in the same plane as the area to be treated and tightly secured to a holder (6), a guide sleeve (11) for the surgical instrument (1) being affixed to the bottom of holder (6) via a vertical joint element (6a), the center of said sleeve simultaneously representing an invariant point (P), and the size of the area to be treated being determined by selecting the radius c on the disk (4c) and an adjustable distance d between the center of disk (4c) and the center of guide sleeve (11).

6. The surgical set of instruments according to claim 5, **characterized in that** the guide element (4) in guide device (2) has a graduation.

7. The surgical set of instruments according to any of claims 1 to 6, **characterized in that** the set additionally comprises a tissue applicator (3) for applying tissue on a defect to be covered, consisting of a holder (12) for the tissue and a tube shaft (13), the holder (12) being arranged optionally rotatably and/or pivotably in a bearing in tube shaft (13).

8. The surgical set of instruments according to claim 7, **characterized in that** the tissue applicator (3) has a forked holder (12a) which is rotatably arranged in a bearing in accommodation element (14) and may optionally be angled relative to the tube shaft (13).

9. The surgical set of instruments according to claim 8, **characterized in that** a link joint (15) is present for angling between tube shaft (13) and accommodation element (14).

10. The surgical set of instruments according to claim 7, **characterized in that** the tissue applicator (3) has a holder (12b) comprised of hooks arranged pivotably in the tube shaft (13).

11. The surgical set of instruments according to claim 10, **characterized in that** the tissue applicator also has a pivotable mandrel (12c) for putting up the tissue.

## Revendications

1. Trousse d'instruments chirurgicaux comprenant un instrument chirurgical (1) et un dispositif de guidage (2) pour le traitement orthogonal des surfaces de cartilage, de tissu osseux ou de tissu corporel, **caractérisée en ce que**, dans le dispositif de guidage (2), il y a un élément de guidage (4) destiné au guidage circulaire de l'instrument chirurgical (1) et un élément de guidage (5) orientable sur un plan horizontal et les deux éléments de guidage (4,5) sont reliés par un support (6), dans lequel l'élément de guidage (4) tournant sur un axe circulaire est disposé de telle sorte que le rayon c du plan de guidage formé par le guidage circulaire de l'instrument (1) est supérieur au rayon du plan de guidage circulaire formé par l'élément de guidage orientable horizontalement et l'instrument chirurgical (1) est guidé de manière excentrique à travers ce plan de guidage, de telle sorte qu'il passe par un point invariant (P) en dessous de l'élément de guidage (5) tournant sur un axe horizontal et que le rayon a de la surface à traiter avec l'instrument chirurgical (1) est ajustable en modifiant l'intervalle d entre les deux éléments de guidage (4,5), selon l'équation

$$a : b = c : d,$$

dans laquelle b représente la distance entre le point invariant (P) et le centre de la surface à traiter.

2. Trousse d'instruments chirurgicaux selon la revendication 1, **caractérisée en ce que**, dans le dispositif de guidage (2), l'élément de guidage (4) tournant sur un axe circulaire est une bague (4a), l'élément de guidage (5) tournant sur un axe horizontal se compose d'un bague (5a) et d'une sphère ou d'une hémisphère tournant sur des roulements à l'intérieur pourvue d'un alésage excentrique, au travers duquel l'instrument chirurgical passe, les deux bagues (4a, 5a) sont reliées l'une avec l'autre par un élément de jonction (6a) et la bague inférieure (5a) est attachée à une hauteur réglable à l'élément de jonction par le support (6).

3. Trousse d'instruments chirurgicaux selon la revendication 1, **caractérisée en ce que**, dans le dispositif de guidage (2), l'élément de guidage (5) tournant autour d'un axe horizontal est la bague intérieure (5c) d'un roulement, dans lequel une demi-coupelle (6b) est disposée de manière à pivoter et perpendiculairement au plan de la bague (5c), à l'extrémité inférieure, positionnée dans le roulement, de laquelle, est fixé un manchon de réception (5d) pour l'instrument chirurgical (1) par un élément de jonction (5e) de telle sorte que l'instrument chirurgical (1) passe de manière excentrique au travers de la bague intérieure (5c) du roulement et qu'à l'autre extrémité, un manchon de réception (4b) pour l'instrument chirurgical (1) est fixé à un élément de fixation à longueur réglable (8) par un élément de jonction (4d), de telle sorte que le rayon c du plan de guidage formé par le guidage circulaire de l'instrument (1) est la distance normale du centre du manchon de réception (4b) à la ligne médiane de la demi-coupelle (6b).

4. Trousse d'instruments chirurgicaux selon une des revendications 1 à 3,
**caractérisée en ce**
**qu'**un des éléments de guidage (4) ou (5) comporte une graduation.

5. Trousse d'instruments chirurgicaux comprenant un instrument chirurgical (1) et un dispositif de guidage (2) pour le traitement des surfaces de cartilage, de tissu osseux ou de tissu corporel sur un plan unique avec les surfaces à traiter,
**caractérisée en ce**
**qu'**elle comporte un instrument chirurgical (1) doté d'une tête coudée et d'un boulon de guidage (10) situés sur le manche ou la poignée de l'instrument, et que le dispositif de guidage (2) comporte un élément de guidage circulaire (4), sous forme d'une rondelle (4c) mobile autour d'un axe comportant des alésages (9) excentriques, disposés de manière hélicoïdale, destinés à l'enfichage facultatif d'un boulon de guidage (10) disposé sur le manche ou la poignée de l'instrument, dans lequel la distance entre l'alésage respectif (9), dans lequel le boulon de guidage (10) est enfiché, et le centre de la rondelle (4c) est désignée comme le rayon c et cette rondelle (4c) se trouve sur le même plan que la surface à traiter et est assemblée fixement avec un support (6), dans laquelle un manchon de guidage (11) pour l'instrument chirurgical (1) est fixé par l'intermédiaire d'un élément de jonction vertical (6a) à l'extrémité inférieure du support (6), dont le centre représente en même temps un point invariant (P), et la dimension de la surface à traiter est déterminée par la sélection du rayon c sur la rondelle (4c) et une distance réglable d entre le centre de la rondelle (4c) et le centre du manchon de guidage (11).

6. Trousse d'instruments chirurgicaux selon la revendication 5,

**caractérisée en ce**
**que** l'élément de guidage (4) situé dans le dispositif de guidage (2) comporte une graduation.

7. Trousse d'instruments chirurgicaux selon une des revendications 1 à 6,
**caractérisée en ce**
**qu'**elle comporte en plus un applicateur de tissu (3) destiné à appliquer un tissu sur un défaut à recouvrir, qui est constitué d'un support (12) pour le tissu et d'un manche de tube (13), dans lequel le support (12) tourne sur des paliers dans le manche de tube (13) éventuellement de manière à être mobile autour d'un axe et/ou à pouvoir être élongé.

8. Trousse d'instruments chirurgicaux selon la revendication 7,
**caractérisée en ce**
**que** l'applicateur de tissu (3) possède un support (12a) fourchu qui tourne autour d'un axe sur des paliers dans un élément de logement (14) qui peut éventuellement être articulé vis à vis du manche de tube (13).

9. Trousse d'instruments chirurgicaux selon la revendication 8,
**caractérisée en ce qu'**elle comporte un assemblage articulé (15) pour le pliage entre le manche de tube (13) et l'élément de logement (14).

10. Trousse d'instruments chirurgicaux selon la revendication 7,
**caractérisée en ce que**
l'applicateur de tissu (3) possède un support (12b) composé de pattes, qui sont disposées de manière à pouvoir être élongées dans le manche de tube (13).

11. Trousse d'instruments chirurgicaux selon la revendication 10,
**caractérisée en ce que**
l'applicateur de tissu comporte en plus un mandrin (12c) pouvant être élongé, pour serrer le tissu.

Fig.1

$$\frac{a}{b} = \frac{c}{d}$$

Fig.2

Fig.3

Fig.5

17   12a

14  15  13

16

3

NiTi

EP 0 967 935 B1

14

Fig.6

EP 0 967 935 B1

EP 0 967 935 B1

Gewebe

12c

12b

3

13

Fig.7

1

4c

10

6c

6d

18

FiG. 8.